Europäisches Patentamt

European Patent Office

Office européen des brevets

(11) Publication number: **0 269 435**
**A2**

(12) **EUROPEAN PATENT APPLICATION**

(21) Application number: **87310410.3**

(22) Date of filing: **25.11.87**

(51) Int. Cl.⁴: **B 01 J 14/00**
G 01 N 27/46, A 61 L 2/18,
A 61 L 2/24

(30) Priority: **27.11.86 GB 8628441**

(43) Date of publication of application:
**01.06.88 Bulletin 88/22**

(84) Designated Contracting States:
**CH DE ES FR GB IT LI NL SE**

(71) Applicant: **UNILEVER PLC**
**Unilever House Blackfriars P.O. Box 68**
**London EC4P 4BQ (GB)**

(84) Designated Contracting States: **GB**

(71) Applicant: **UNILEVER NV**
**Burgemeester s'Jacobplein 1 P.O. Box 760**
**NL-3000 DK Rotterdam (NL)**

(84) Designated Contracting States:
**CH DE ES FR IT LI NL SE**

(72) Inventor: **Lips, Leonardus**
**Goebelstrasse 40**
**Emmerich (DE)**

(74) Representative: **Van Gent, Jan Paulus et al**
**Unilever N.V. Patent Division P.O. Box 137**
**NL-3130 AC Vlaardingen (NL)**

(54) **Disinfectant dilution.**

(57) In a method and apparatus for diluting a reaction product, particularly a peracid for sanitizing purposes, reactants from an inlet 75 are reacted in a reaction vessel 3,73 provided with means 13 for impeding their ingress to minimise turbulence as they mix with the body of liquid within the reactor. An outlet 11 is positioned remote from the impeding means to remove liquid generally having the longest residence time in the chamber. Dilution of the reaction product is controlled in accordance with the output of amperometric electrochemical sensor(s) 131 - 141. Preferably, the reaction product is a peracid especially performic and the sensor has a gold working electrode at around zero volts.

EP 0 269 435 A2

Bundesdruckerei Berlin

**Description**

## DISINFECTANT DILUTION

The present invention relates to a chemical reactor particularly suited, although not exclusively, to the preparation of disinfectant products. The invention further extends to apparatus and methods for controlled dilution of said disinfectants to render them suitable for use.

U.K. Patent Application No. 8531384, unpublished at the priority date of the present application, describes an industrial method for sanitising surfaces, by preparing performic acid and applying it at an effective concentration to the surface, immediately or within a time period before degradation renders it ineffective.

The latter method is an advance over the previously known use of, for example, peracetic acid. Performic and peracetic acids are examples of peracids generally, which conventionally are prepared by reaction of hydrogen peroxide and the appropriate organic acid, optionally in the presence of a suitable catalyst such as a strong acid. Such peracids have an inherent tendency to instability which means that their effective working lifetime is limited. This is particularly so with performic acid. Therefore, the method related in the aforementioned patent application requires preparing the performic acid and using it immediately or within a relatively short time afterwards. However, such preparation and prompt use would be highly desirable with use of any peracid intended for such purposes, or indeed with any unstable substance for like or comparable purposes.

According to one aspect of the present invention, we have devised a chemical factor, suitable for such preparation, comprising a reaction chamber having a reactant inlet and a reaction product outlet, the reaction chamber being provided with means for impeding ingress of reactants to minimise turbulence as they mix with any body of liquid already within the reactor, said outlet being positioned remote from said means for impeding ingress.

This reactor is suitable for either continuous or batch operation. In the case of batch operation, the reactants entering the reaction chamber impinge on the means for impeding their ingress and then mix with the body of liquid without substantially disturbing it. Thus, the concentration of reaction product at any time will be greatest adjacent the remote outlet. The same holds for continuous operation, except that the reactant:product gradient will be established in a state of dynamic equilibrium. The outlet then serves to remove liquid which has generally had the longest residence time in the reaction chamber, i.e. generally having the highest concentration of reaction product. For this reason, the advantages of the reactor are especially important in continuous operating mode.

The means for impeding ingress of reactants may be any physical obstruction which prevents the majority of inflowing reactants from impinging directly on the surface of the body of liquid but delays or diverts it so it can then be introduced to the liquid body with minimum turbulence. However, we have found it particularly advantageous to use for this purpose, a float arranged to float on top of the body of liquid with a path being provided for allowing reactants past the float into said liquid body.

It is especially preferred to provide the float with a circumferential rim for retaining liquid on top of the float. Not only does this provide a "reservoir" for the reactants after they have entered so that they can intimately inter-mix and begin reacting, but it absorbs much of the kinetic energy in the entering stream of reactants. It is then preferred for the path allowing the reactants into the body of liquid to be provided in the form of a gap between the rim and the inside of the reaction chamber. Thus, the liquid retained within the rim gently dribbles over the side, as with a weir. It will be appreciated that it is most convenient for the internal profile of the reaction chamber, and also the float, to be substantially circular. However, it should be noted that other means are possible for allowing reactants past the float into the body of the liquid, for example one or more small holes in the float surface, or by making at least a portion of the float out of a liquid permeable material.

Further features of the present invention are concerned with the actual dilution process, for diluting the reaction products prior to use in sanitisation.

Thus forms of the present invention provide apparatus and a method for preparing a diluted reaction product, by:-

    a) effecting a reaction between two or more reactants, desirably in a reactor as set forth above, to form a reaction product;

    b) diluting the reaction product;

    c) monitoring the concentration of reaction product after dilution, by using an electrochemical sensor; and

    d) controlling the dilution of the reaction product in accordance with the output of said sensor.

One recently known system employing peracetic acid as sanitizer employs a redox electrochemical measurement as part of a control system governing the dilution of sanitizer. However, this suffers from the disadvantage that the sensor can be sensitive to 'interfering' species in the liquid, either giving a less than perfect measurement of peracid concentration, or requiring addition of another chemical to the liquid in order to suppress the interference. We have found that we can overcome this problem by use of an amperometric technique. This is generally applicable to monitoring concentration of unstable reaction products in dilution operations.

It is a preferred feature of this invention to utilise an amperometric electrochemical sensor, which desirably has a working electrode, a counter electrode and a reference electrode.

Preferably, a second amperometric electrochemical sensor of substantially identical construction is used to monitor the peracid concentration in the reaction product prior to dilution, the output of the two sensors then being used to control the dilution.

Control of the dilution mixture can be achieved either by feedback or by feedforward techniques or a combination of the two. Feedback control would use the concentration of the diluted solution to control the addition of diluent. Feedforward control would use the concentration of the reaction mixture to predict the required amount of diluent. The combination feedforward-feedback technique would in addition use the dilute product measurement to trim the diluent prediction calculation.

In one preferred embodiment the reaction is between an organic acid and hydrogen peroxide, to form a peracid. Most preferably, this is the reaction between formic acid and hydrogen peroxide, optionally in the presence of a small amount of catalyst such as a strong mineral acid (e.g. sulphuric) to form performic acid.

When the peracid in question is peracetic acid or performic acid, it is especially preferred that the working electrode is made of gold and operated at a potential of around zero volts. To be meaningful in thermodynamic terms, such potentials should be expressed relative to a nominal reference electrode. In this case the preferred reference is the Ag/AgCl electrode.

The present invention will now be illustrated by way of the following exemplary and non-limiting embodiments, with reference to the accompanying drawings in which:-

     Figure 1 shows in axial cross-section, a reaction vessel according to the present invention.

     Figure 2 shows a cross-sectional view of an optional mixing chamber for mixing of reactants prior to their introduction into the mixing tank shown in Figure 1.

     Figure 3 shows a schematic view of a PFA reactor unit incorporating the reaction vessel of Figure 1.

     Figure 4 shows a schematic view of the reactor unit of Figure 3, incorporated in a CIP system.

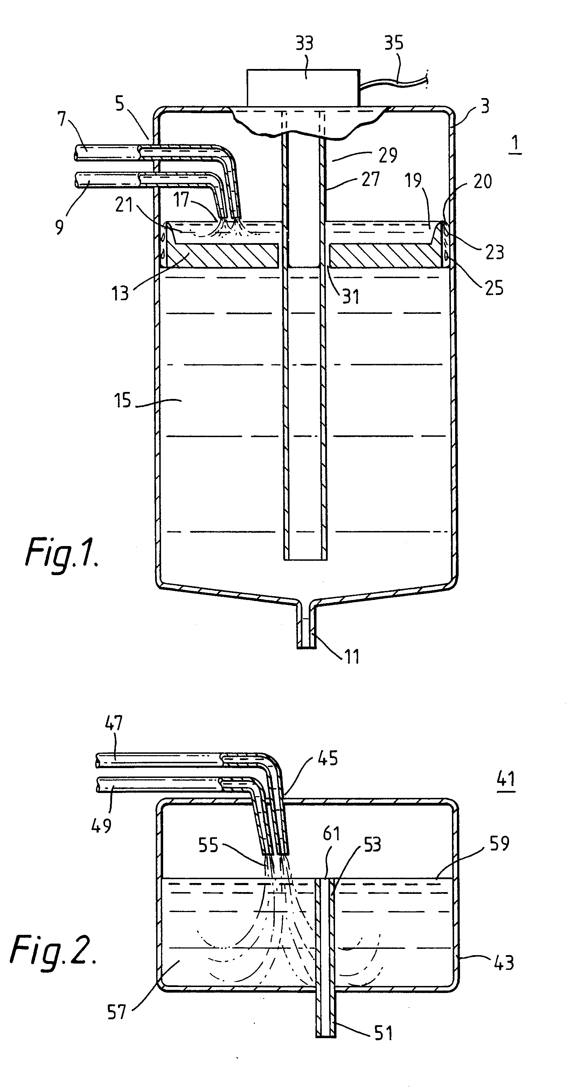

Figure 1 shows a reaction vessel 1 comprising a substantially cylindrical reaction chamber 3. The chamber has an inlet in the upper region of its curved wall, and comprises a pair of reactant inlet conduits 7, 9. An outlet 11 for the reaction product is situated at the lowermost end of the chamber. A substantially circular disc-shaped float 13 of high density polyethylene floats on top of the body of liquid 15 which comprises the reacting liquor.

As reactant 17 (hydrogen peroxide and formic acid) enter the chamber via inlet conduits 7, 9, they impinge on the float and fill the sunken portion 19 of the float, bounded by a raised rim 20. The liquid reactants then spill over the side at 23, and enter the inside wall of the reaction chamber. Inevitably, a small amount of liquid will also spill through the gap defined by central hole 31 in the float and the measurement tube 27 of a level sensor 29 of a conventional kind.

The level sensor also comprises a measurement head 33 providing an electrical output on cable 35, in accordance with the current level of the surface of the body of liquid and/or the float within the chamber. The output of the sensor is used to control the liquid flow rate of the incoming reactants and the outflow of reaction product at 11. This product contains a substantial proportion of performic acid (PFA).

The principle of operation of this reactor is that when the flowrates have been set correctly, there exists a concentration gradient of peracid in the body of the liquid, from a minimum in the region of the float, to a maximum in the region of the outlet.

As shown in Figure 1, the reactants enter via separate conduits and primary mixing takes place in the sunken portion of the float. However, it may be advantageous to pre-mix the reactants in a separate chamber of the type shown in Figure 2. Here, a mixer 41 comprises a mixing tank 43 having an inlet 45 comprising a pair of conduits 47, 49. An outlet 51 is provided in the form of a tube having a section 53 protruding inside the tank. The incoming reactants 55 mix within the body of liquid 57, the surface of which assumes the level of the tip 61 of the protruding section of the outlet tube which is connected direct to the reaction vessel.

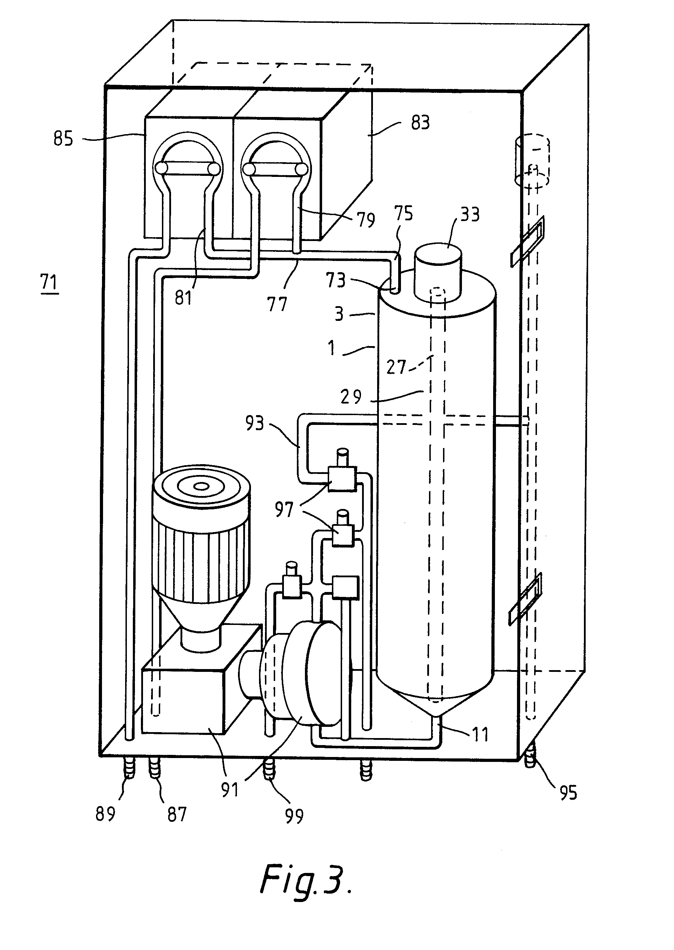

Figure 3 shows a reactor unit 71, incorporating a reaction vessel 1 of the kind shown in Figure 1, in this case having an inlet 73 in the form of a single conduit 75 which is fed by a simple mixer 77. The mixer combines the outputs 79, 81 of peristaltic pumps 83, 85 respectively. These in turn obtain supplies of the reactants hydrogen peroxide and formic acid from inlets 87, 89.

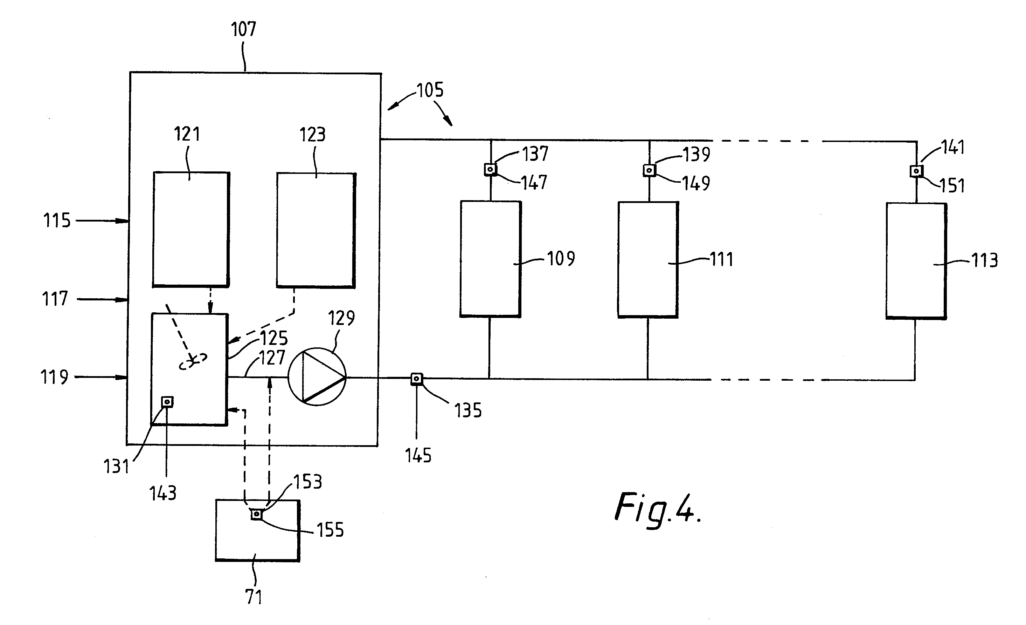

The outlet of the reactor feed pump 91 where it is diluted with water from pipe 93, connected to water supply 95. The degree of dilution is controlled by automatic valves 97 in a manner to be described further hereinbelow. The diluted outlet supplies dilute PFA from outlet 99 to a CIP system, for example as shown in Figure 4.

Here, a CIP system 105 comprises a CIP unit 107 connected to first, second and to n th equipment lines 109, 111, 113 respectively. The CIP system is connected to the PFA reactor unit 71, shown previously in Figure 3. The CIP unit itself is provided with inputs 115, 117, 119 of water, power and detergent. The water and detergent are stored in reservoirs 121, 123 and are combined in mixing tank 125. The performic acid may be added direct to the mixing tank or to its outlet 127.

The mix of water, detergent and PFA is impelled by pump 129 into the equipment lines.

Where regulations would not permit direct coupling of such a reactor unit to the water supply, reactant can be mixed before entering the reaction chamber by dropping into a funnel. Water is then only connected to the reactor for flushing between operations and all dilution of the reaction product occurs within the CIP system itself.

For control of the disinfection, PFA sensors may be provided in one or more of a number of places, but preferably all of these. Thus, a sensor may be provided in the mixing tank at 131, the pump outlet at 135 and in the equipment lines at 137, 139 and 141.

These sensors are three-electrode amperometric devices which may be of a form which will be known to those skilled in the art. The working electrode is gold and is arranged by suitable circuitry to operate at a voltage of substantially zero volts. The reference electrode is silver/silver chloride and the counter electrode is of platinum. The outputs 143, 145, 147, 149, 151 are fed to appropriate electronic control circuitry and used to control the degree of dilution by operation of the automatic mixing valves 97, shown in Figure 3. The manner of this control also will be apparent to those skilled in the art.

Comparative measurements, for the purpose of improved control, may be made using identical sensors. Thus, for example, a sensor 153 may be provided on the diluted PFA outlet of the reactor unit, again the output 155 being fed to the control circuitry. Additionally or alternatively, another sensor may be placed in the reactant outlet 11 (see Figure 3) of the PFA reactor.

**Claims**

1. A chemical reactor comprising a reaction chamber having a reactant inlet and a reaction product outlet, the reaction chamber being provided with means for impeding ingress of reactants to minimise turbulence as they mix with any body of body of liquid already within the container and said outlet being positioned remote from said means for impeding ingress.

2. A reactor according to claim 1, wherein the means for impeding ingress is a float arranged to float on top of said body of liquid to cover at least a major proportion thereof, a path being provided for allowing the reactants past the float into the body of liquid.

3. A reactor according to claim 2, wherein the float has a circumferential rim for retaining liquid on top of the float and the path for allowing reactants past the float is provided by a gap between the rim and the inside of the reaction chamber.

4. A reactor according to any preceding claim, wherein a level sensor is provided for controlling the rate of ingress of reactants and outflow of reaction product.

5. A reactor according to claim 4, when dependent on claim 2 or claim 3, wherein part of the level sensor protrudes through a hole in the float.

6. Apparatus for production and dilution of a reaction product, comprising a reactor according to any one of the preceding claims for preparing the reaction product, means for diluting the reaction product, an electrochemical sensor for measuring reaction product concentration, and a controller arranged to control the dilution of the reaction product in accordance with the output of the sensor.

7. Apparatus according to claim 6, for controlling the dilution of a peracid, wherein the sensor is an amperometric electrochemical sensor which comprises a counter electrode, a reference electrode and a gold working electrode arranged to operate at a potential of around zero volts relative to the reference electrode.

8. Apparatus according to claim 6 or claim 7, wherein the sensor is arranged to measure the reaction product concentration after dilution.

9. Apparatus according to claim 8 connected to a system utilizing the reaction product, and wherein one or more additional sensors are located to measure the reaction product concentration at points in the system.

10. Apparatus according to claim 8 or claim 9, wherein a sensor substantially identical to the other(s) is positioned to measure the reaction product concentration prior to dilution and its output is used for comparative control.

11. A method of preparing a diluted reaction product, the method comprising:-

   a) effecting a reaction between two or more reactants, in a reactor according to any one of claims 1 to 5, to form a reaction product;

   b) diluting the reaction product;

   c) monitoring the concentration of the reaction product after dilution, by using an electrochemical sensor;
   and

   d) controlling the dilution of the reaction product in accordance with the output of said sensor.

12. A method according to claim 11, wherein the reaction is between an organic acid and hydrogen peroxide to form a peracid and wherein the electrochemical sensor is an amperometric sensor which comprises a counter electrode, a reference electrode and a gold working electrode arranged tooperate at a potential of around zero volts relative to the reference electrode.

13. A method according to claim 12, wherein the organic acid is acetic acid or formic acid and the peracid is peracetic acid or performic acid respectively.

14. A method according to claim 13, wherein the peracid is performic acid.

0269435

Fig.1.

Fig.2.

*Fig.3.*

Fig.4.

0269435